(19) Europäisches Patentamt
European Patent Office
Office européen des brevets

(11) **EP 4 553 498 A1**

(12) **EUROPEAN PATENT APPLICATION**
published in accordance with Art. 153(4) EPC

(43) Date of publication:
**14.05.2025 Bulletin 2025/20**

(21) Application number: **23835589.5**

(22) Date of filing: **06.07.2023**

(51) International Patent Classification (IPC):
*G01N 33/15* (2006.01)      *G06Q 50/02* (2024.01)

(52) Cooperative Patent Classification (CPC):
**A01G 7/00; G01N 33/15; G06Q 50/02**

(86) International application number:
**PCT/JP2023/025087**

(87) International publication number:
**WO 2024/010058 (11.01.2024 Gazette 2024/02)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **07.07.2022  JP 2022109418**

(71) Applicant: **Agri Smile, Inc.**
**Tokyo 101-0052 (JP)**

(72) Inventors:
• **HAYASHI, Daisuke**
  **Tokyo 101-0052 (JP)**
• **OHDO, Yukiko**
  **Tokyo 101-0052 (JP)**

(74) Representative: **Hoffmann Eitle**
**Patent- und Rechtsanwälte PartmbB**
**Arabellastraße 30**
**81925 München (DE)**

(54) **INFORMATION PROCESSING METHOD, RECORDING MEDIUM, AND INFORMATION PROCESSING DEVICE**

(57)    Provided is an information processing method, wherein an information processing device executes: acquiring measurement values of one or a plurality of factor items related to a biostimulant effect factor of a plant from the plant to which a material with a function of enhancing tolerance to abiotic stress has been applied; weighting each acquired measurement value of each factor item using each weight; and calculating an evaluation value of the material by inputting each measurement value weighted by each of the weights into a function related to evaluation of the material.

**Fig. 9**

EP 4 553 498 A1

## Description

### Technical Field

The present invention relates to an information processing method, a recording medium, and an information processing device.

### Background Art

Conventionally, there have been materials that may be referred to as biostimulants, which have the function of enhancing tolerance to abiotic stress in plants, and attempts have been made to apply these materials to plants to promote their growth and development and to improve crop yields or quality (see, for example, Patent Literature 1).

### Citation List

### Patent Literature

Patent Literature 1: Patent Publication JP-A-2022-66901

### Summary of Invention

### Technical Problem

However, the conventional technology has difficulty in evaluating materials applied to plants. For example, depending on used cultivation environments or the conditions of the physiological cycles of plants, appropriate material concentration or spraying methods differ. However, the functions of the materials have not been fully understood, and there are cases where no effects are observed during actual use at production sites even though the materials are applied.

Accordingly, it is an object of the technology of the present disclosure to provide a new mechanism that appropriately evaluates the effects of materials.

### Solution to Problem

An aspect of the present disclosure provides an information processing method, wherein an information processing device executes: acquiring measurement values of one or a plurality of factor items related to a biostimulant effect factor of a plant from the plant to which a material with a function of enhancing tolerance to abiotic stress has been applied; weighting each acquired measurement value of each factor item using each weight; and calculating an evaluation value of the material by inputting each measurement value weighted by each of the weights into a function related to evaluation of the material.

### Advantageous Effect of Invention

According to the present disclosure, it is possible to appropriately evaluate the effects of materials.

### Brief Description of Drawings

[Figure 1] Figure 1 is a diagram showing an example of the configuration of an information processing system according to an embodiment of the present disclosure.

[Figure 2] Figure 2 is a diagram showing an example of the configuration of an information processing device according to an embodiment of the present disclosure.

[Figure 3] Figure 3 is a diagram showing an example of the configuration of an information processing device according to an embodiment of the present disclosure.

[Figure 4] Figure 4 is a diagram showing an example of each factor item information (part 1) in the present disclosure.

[Figure 5] Figure 5 is a diagram showing an example of each factor item information (part 2) in the present disclosure.

[Figure 6] Figure 6 is a diagram showing an example of the weighting of each factor item and the calculation of evaluation values (case 1) in the present disclosure.

[Figure 7] Figure 7 is a diagram showing an example of the weighting of each factor item and the calculation of evaluation values (case 2) in the present disclosure.

[Figure 8] Figure 8 is a diagram showing an example of grading in the present disclosure.

[Figure 9] Figure 9 is a sequence diagram showing an example of the evaluation processing performed by the information processing device according to an embodiment of the present disclosure.

[Figure 10] Figure 10 is a sequence diagram showing an example of the grading processing performed by the information processing device according to an embodiment of the present disclosure.

### Description of Embodiments

With reference to the accompanying drawings, preferred embodiments of the disclosed technology will be described. Note that in each figure, components indicated by the same reference symbols have the same or similar configurations.

[Embodiment]

<System>

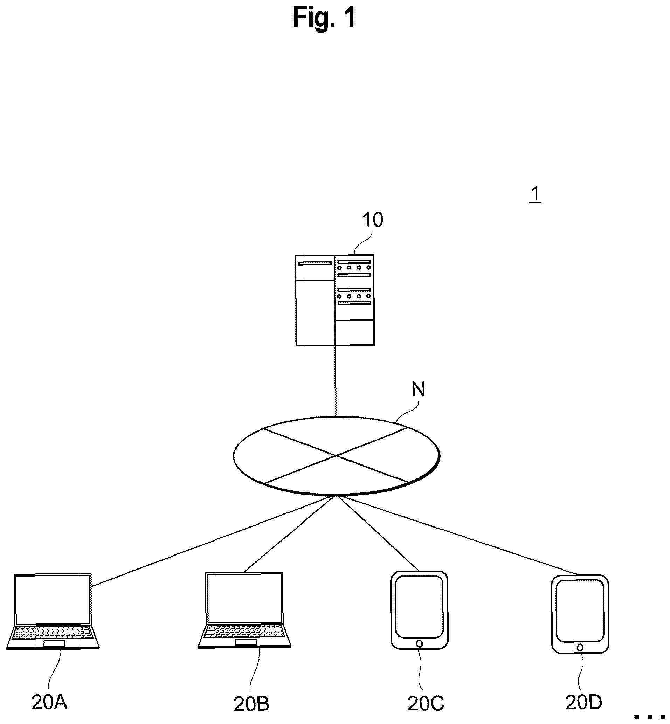

Figure 1 is a diagram showing an example of the configuration of an information processing system 1 according to an embodiment of the present disclosure. As shown in Figure 1, the information processing system 1 includes an information processing device 10 and information processing devices 20A, 20B, 20C, and 20D (also collectively referred to as an "information processing device 20"), and the information processing devices 10 and 20 can transmit and receive data to and from each other via a network N.

Here, the material to be evaluated in the technology of the present disclosure will be described. The material to be evaluated is an agricultural material also referred to as a biostimulant (hereinafter referred to as a "BS"), which is a biostimulant agent including various substances or microorganisms that provide improved physiological conditions to plants or soil. The material is one capable of having favorable influence on plants in terms of their health, stress tolerance, yield and quality, post-harvest conditions and storage, or the like, by utilizing the natural power inherent in plants or their surrounding environments.

The BS is generally made from natural ingredients, extracts derived from animals and plants, metabolic products originating from microorganisms, or the like. The BS may also be a single substance or a composite of these natural ingredients, extracts and/or metabolic products . Furthermore, unlike agricultural chemicals, the BS includes materials that have the effect of alleviating abiotic stress.

Examples of the effects of the BS include suppression of active oxygen, activation of photosynthesis, promotion of flowering and fruit set, control of transpiration, regulation of osmotic pressure, an improvement in rhizosphere environment, an increase in root mass, an improvement in root activity, or the like. However, the BS does not possess all of these effects.

Next, the outline of a method for evaluating the effects of the BS applied to plants, which is realized by the information processing system 1 shown in Figure 1, will be described. For example, the evaluation method of the present disclosure utilizes items that can serve as biostimulant effect factors from the viewpoint of the physiological functions of plants, and applies weighting based on the degree of influence of these items to calculate indices for evaluating the effects of the material.

As a result, for materials such as BS whose mechanisms of action have not been fully understood and whose effects remain unclear until used, it becomes possible to evaluate the effects of these materials by applying weighting based on the degree of influence of factor items that can serve as biostimulant effect factors.

The above information processing devices 10 and 20 shown in Figure 1 are, for example, personal computers, mobile terminals such as smartphones, tablet terminals, server devices, or the like, each of which may be referred to as an n-th information processing device (where n = 1, 2, 3 ...) in order to distinguish them from one another.

Furthermore, the information processing device 10 receives the measurement values (including analysis values) of each factor item of materials to calculate their evaluation values, classify the materials into groups on the basis of their effects, or grade the materials. The information processing device 20 acquires measurement values by measuring or analyzing the factor items of the materials, displays the measurement values on a screen, and transmits the measurement values to the information processing device 10. Furthermore, the information processing device 10 may analyze or measure each of the factor items processed by the information processing device 20.

<Configuration>

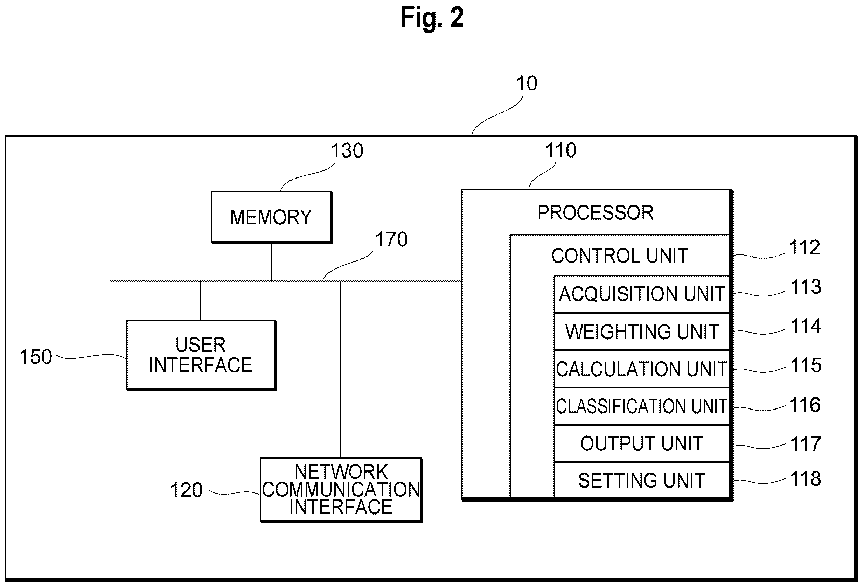

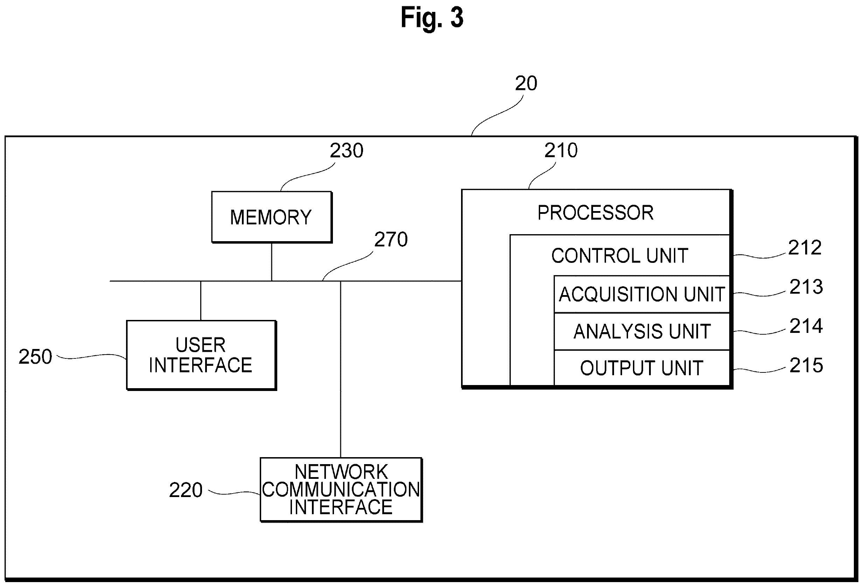

Figure 2 is a diagram showing an example of the configuration of the information processing device 10 according to an embodiment of the present disclosure. Figure 3 is a diagram showing an example of the configuration of the information processing device 20 according to an embodiment of the present disclosure. Hereinafter, the processing of each device will be described using a specific example of evaluation indices for a material with the function of enhancing tolerance to abiotic stress.

The information processing device 10 includes one or a plurality of processors (CPU: Central Processing Unit) 110, one or a plurality of network communication interfaces 120, a memory 130, a user interface 150, and one or a plurality of communication buses 170 used to connect these components to each other.

The user interface 150 includes a display and input devices (a keyboard and/or a mouse, or any other pointing device, or the like), but it is not necessarily required to be provided in the information processing device 10. When provided, the user interface 150 may also be connected as an external device.

The memory 130 is, for example, a high-speed random access memory (main storage device) such as DRAM, SRAM, or other random access solid-state storage devices. Furthermore, the memory 130 may also be a non-volatile memory (auxiliary storage device) such as one or a plurality of magnetic disk storage devices, optical disk storage devices, flash memory devices, or other non-volatile solid-state storage devices.

Furthermore, the memory 130 may also be a non-transitory computer-readable recording medium that stores programs or the like. Additionally, the memory 130 may be one of a main storage device (memory) and an auxiliary storage device (storage), or it may include both devices.

Furthermore, as another example of the memory 130, one or a plurality of storage devices installed remotely from the processor 110 may be provided. In one embodiment, the memory 130 stores a program executed by the processor 110, modules, data structures, or their subsets.

The memory 130 stores data to be used by the information processing system 1. For example, the memory 130 stores information related to materials, one or a plurality of factor items related to biostimulant effect factors of plants, measurement values of each factor item, functions used to evaluate materials, evaluation values of each material, criteria for classifying materials using evaluation values, and information related to grading standards.

The processor 110 configures a control unit 112, an acquisition unit 113, a weighting unit 114, a calculation unit 115, a classification unit 116, an output unit 117, and a setting unit 118 by executing the program stored in the memory 130.

The control unit 112 controls processing related to the evaluation of materials. The control unit 112 controls the processing performed by the acquisition unit 113, the weighting unit 114, the calculation unit 115, the classification unit 116, the output unit 117, and the setting unit 118.

The acquisition unit 113 acquires the measurement values of one or a plurality of factor items related to biostimulant effect factors of a plant from the plant to which a material (for example, a BS) with the function of enhancing tolerance to abiotic stress has been applied. For example, the acquisition unit 113 may acquire measurement values via the network communication interface 120, which are obtained from processing in which each information processing device 20 measures or analyzes the data of each factor item. Furthermore, for factor items obtained from actual measurements of a plant or the like, the acquisition unit 113 may acquire input measurement values when a user or the like inputs the measurement values via the user interface 150. Note that "acquires measurement values from a plant to which a BS has been applied" includes acquiring values via the network communication interface 120, which are obtained when the plant to which the BS has been applied is measured or analyzed as described above, as well as actually measuring the plant itself and receiving the measurement values via the user interface 150, or the like.

The weighting unit 114 weights each measurement value of each factor item acquired by the acquisition unit 113 using each weight. For example, a weight is set for each factor item on the basis of priority. For example, a weight is assigned to each factor item on the basis of the degree of influence of a BS on yield, the degree of influence on plant growth or stimulation, the sequence of physiological processes in plants, or the like.

The calculation unit 115 inputs each measurement value, to which each weight has been assigned by the weighting unit 114, into a function related to the evaluation of a material such as a BS to calculate the evaluation value of the material. For example, the calculation unit 115 may also calculate the total sum of each weighted measurement value as the evaluation value E of a material (Formula 1).

$$E = \Sigma wi \times si \dots \text{Formula 1}$$

i: factor item

wi: weight of factor item i

si: measurement value of factor item i

For example, when the data of the measurement values of each factor item for a material is gathered to a designated value or more, the calculation unit 115 may calculate an evaluation value through a machine learning model that receives the measurement values of each factor item to predict the evaluation value. As an example, the calculation unit 115 may calculate an evaluation value through a trained model that has performed supervised learning with training data including the measurement values of each factor item and annotated evaluation values. Furthermore, the calculation unit 115 may calculate the standard deviation as an example of an evaluation value.

The output unit 117 may output the evaluation value calculated by the calculation unit 115 for display on a display or the like in association with a material.

Through the above processing, by using the measurement values of factor items that can serve as biostimulant effect factors induced by a material such as a BS and applying weighting on the basis of the degree of influence of the factor items, it becomes possible to appropriately evaluate the effects of the material. Furthermore, through the above processing, it becomes possible to provide appropriate evaluation indices to solve problems such as the inability to measure or difficulty in identifying the effects of a material, for a material such as a BS with the function of enhancing tolerance to abiotic stress.

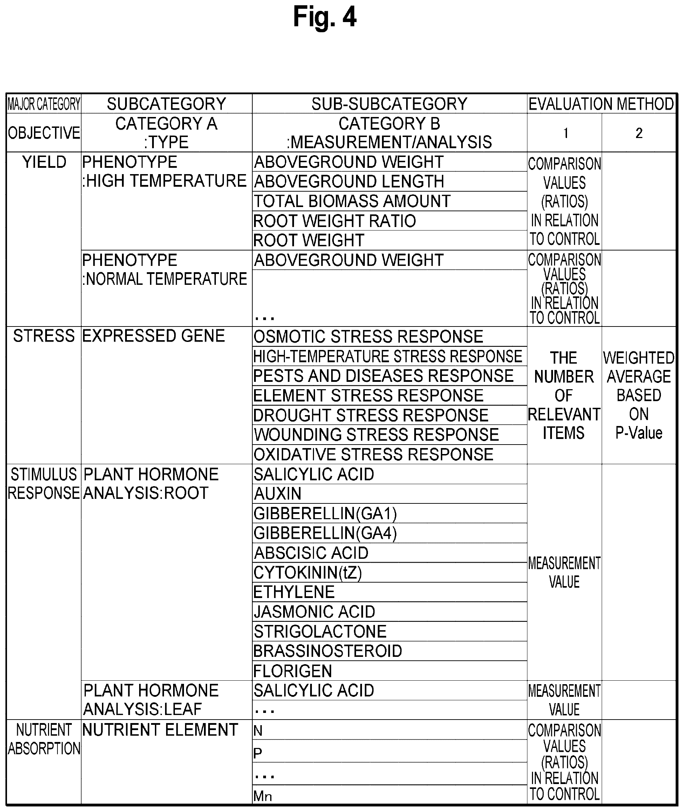

Furthermore, each factor item may include at least one of a plant phenotype, an absorbed nutrient element, hormone analysis in response to stimuli, an expressed gene, or the like. These factor items are those that can be analyzed or measured in a laboratory or the like. Note that each factor item analyzed or measured in a laboratory will be described later with reference to Figure 4.

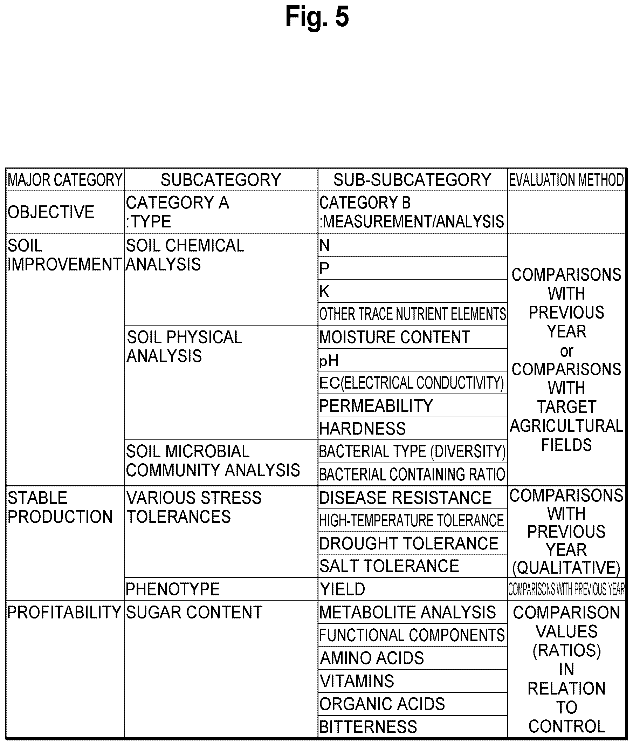

Furthermore, each factor item may also include at least one of soil chemical analysis, soil physical analysis, soil microbial community analysis, stress tolerance, or metabolite analysis. These factor items are those for which soil sampled from an actual agricultural field can be analyzed or measured by a local sensor, or brought back to a laboratory or the like to be analyzed or measured. Each factor item analyzed or measured in an actual agricultural field will be described later with reference to Figure 5. Note that each factor item used for evaluation may include combinations of each factor item analyzed or measured in a laboratory and each factor item analyzed or measured in an actual agricultural field.

Furthermore, each factor item may be classified into a plurality of items through segmentation, and a weight may be assigned to each item. For example, one factor item may be classified according to granularity, such as major category, subcategory, and sub-subcategory, i.e., the factor item may be divided into a major category, one or a plurality of subcategories derived from one major category, and one or a plurality of sub-subcategories derived from one subcategory. Furthermore, weights may be assigned to each major category, subcategory, and sub-subcategory. For example, the weight of each sub-subcategory within the same subcategory may be set on the basis of the priority of each sub-subcategory. As a result, it becomes possible to appropriately set weights on the basis of priority, such as the degree of influence on yield, the degree of influence on plant growth or stimuli, the sequence of physiological processes in plants, or the like.

Furthermore, there are cases where a plurality of different materials are applied to a designated plant under different conditions. In this case, the measurement values of each factor item are acquired from each material by the acquisition unit 113. The acquisition unit 113, the weighting unit 114, and the calculation unit 115 execute processing on each measurement value for each material. Finally, the calculation unit 115 calculates the evaluation value of each material for the designated plant. Each material is preferably applied separately under the same environment to serve as a comparison target, but it may also be evaluated under different environments such as different locations and different periods.

When the measurement values of each factor item are acquired from a plurality of materials by the acquisition unit 113, the classification unit 116 classifies each material using the evaluation values of each material. For example, the classification unit 116 may classify each material into groups according to the degree of the effects indicated by the evaluation values, using a known clustering method. Furthermore, the classification unit 116 may classify each material using each evaluation value through a machine learning model that performs clustering.

Through the above processing, it becomes possible to grasp which material is effective when applying various materials to a designated plant. Conventionally, it has not been possible or has been difficult to evaluate the effects of materials because their mechanisms of action have not been fully understood. However, the technology of the present disclosure can provide evaluation indices for materials, enabling the grouping of materials that produce similar effects, the comparison of effects among materials, and the like.

Furthermore, the classification unit 116 may also include classifying each material using evaluation values for each type of raw material. For example, the following types are included as raw material types of the main components of the materials.

　　1. Humic substances, organic acid materials (humic acids, fulvic acids)

　　2. Seaweeds and seaweed extracts, polysaccharides

　　3. Amino acids and peptide materials

　　4. Trace minerals, vitamins

　　5. Microbial materials (Trichoderma species, mycorrhizal fungi, yeast, bacillus subtilis, rhizobia, etc.)

　　6. Others (Functional components derived from plants and animals, microbial metabolites, microbial activators, etc.)

For example, the memory 130 stores the raw material information of materials, in which information (material IDs) for identifying material names or the materials is associated with the raw material type information of the materials. The classification unit 116 refers to the raw material information of the materials and identifies the raw material types of the materials using the material names or material IDs that are classification targets, and classifies the materials for each type of raw material as a first-stage classification. Next, the classification unit 116 classifies the materials using evaluation values for each type of raw material as a second-stage classification. As a result, it becomes possible to classify materials on the basis of their effects for each type of raw material and to specify or recommend more effective materials for each type of raw material. Note that the classification unit 116 may further classify materials using raw material types for each classification group after classifying the materials using the evaluation values.

Furthermore, the acquisition unit 113 may acquire a classification request from the user or the like. The classification unit 116 may extract materials that have evaluation values corresponding to conditions included in this user request and classify the extracted materials. The user request includes, for example, information related to the above raw material type of a material, information related to a specific factor item, information related to the environment of an agricultural field, or the like. As for determining whether the evaluation values correspond to the conditions included in the user request, the evaluation values may be considered to "correspond" to the conditions, for example, when the evaluation values fall within a designated range of the numerical values included in the conditions, or when the evaluation values are above or below a threshold. Furthermore, the numerical values included in the conditions may also be values related to designated factor items, besides values related to the evaluation values of the materials.

For example, when gene analysis items are included in the user request, the classification unit 116 may classify each material using values or evaluation values obtained by weighting measurement values/analysis values for each gene analysis item. The gene analysis items include, for example, high-temperature stress response, osmotic stress response, oxidative stress response, drought stress response, and wounding stress response.

For example, when the acquisition unit 113 receives a user request from a producer regarding the need for tolerance to high-temperature stress, the classification unit 116 may classify materials that have high tolerance to the factor item "high-temperature stress response" (for example, materials whose weighting value for high-temperature stress response is greater than a threshold) and group the classified materials in response to the request. Information including the grouped materials is output to the producer by the output unit 117.

When the evaluation value of a material satisfies a designated condition related to a recommendation, the output unit 117 may output information related to this material and recommendation information. The designated condition includes, for example, a condition that the evaluation value exceeds a threshold. In this case, when the evaluation value calculated by the calculation unit 115 exceeds a designated threshold, the output unit 117 may regard the material as highly effective and output information that recommends the material. As a result, it becomes possible to specify highly-effective materials using the evaluation indices of the materials and to notify the persons concerned of the highly-effective materials. Furthermore, the designated condition may also include a condition related to adaptability to fertilizers used by producers. In this case, the recommendation information can include the information of materials adapted to the fertilizers used by the producers. Furthermore, the designated condition may also include a condition related to stress tolerance. For example, the stress tolerance may be determined on the basis of the result of a comparison between the analysis value for a designated stress response and a threshold. In this case, the recommendation information may include the information of materials that have tolerance to a designated stress response.

Furthermore, the setting unit 118 may grade materials using evaluation values, either for each group classified by the classification unit 116 or on the basis of the result of a comparison between the standard deviations of the evaluation values and thresholds. For example, the setting unit 118 may also assign a rank to each material to indicate the degree of effect. As a result, evaluation results can be assigned to designated materials. Furthermore, the above recommendation information may also include grading ranks.

According to the above processing, it is possible to provide the evaluation indices of materials for a designated plant using the technology of the present disclosure and to notify the effects of the materials using these evaluation indices. Additionally, it is possible to use the evaluation indices as evidence to indicate the effects of the materials, as they are appropriate for this purpose.

Furthermore, an organization that manages the information processing device 10 can assign evaluation indices to existing materials and thus provide evaluation services for materials as a material evaluation organization. In this case, the evaluation values may be used as evidence for evaluation results.

Furthermore, according to the technology of the present disclosure, it is possible to identify highly-effective materials on the basis of appropriate evaluation indices, thereby enabling the grading of materials. Furthermore, the organization that manages the information processing device 10 may also grant a license for a technical method that calculates the evaluation indices.

Figure 3 is a diagram showing an example of the information processing device 20 according to an embodiment of the disclosure. The information processing device 20 includes one or a plurality of processors (e.g., CPU) 210, one or a plurality of network communication interfaces 220, a memory 230, a user interface 250, and one or a plurality of communication buses 270 used to connect these components to each other.

The user interface 250 includes a display and input devices (a keyboard and/or a mouse, or any other pointing device, or the like).

The memory 230 is, for example, a high-speed random access memory (main storage device) such as DRAM, SRAM, or other random access solid-state storage devices. Furthermore, the memory 230 may also be a non-volatile memory (auxiliary storage device) such as one or a plurality of magnetic disk storage devices, optical disk storage devices, flash memory devices, or other non-volatile solid-state storage devices. Furthermore, the memory 230 may also be a non-transitory computer-readable recording medium that stores programs or the like.
Furthermore, the memory 230 may also be one of a main storage device (memory) and an auxiliary storage device (storage), or it may include both devices.

The memory 230 stores the data or programs used by the information processing system 1. For example, the memory 230 stores an application program or the like for a mobile terminal in the information processing system 1.

The processor 210 configures a control unit 212, which controls processing related to the calculation of evaluation indices for a BS on a client side, by executing the program stored in the memory 230. For example, the control unit 212 includes a web browser, an application related to the calculation of evaluation indices, or the like.

The web browser makes it possible to view the web pages of the evaluation indices calculation platform provided by the information processing device 10. The web browser appropriately displays and navigates the web pages and performs the provision of information, the transmission/reception of data, or the like. For example, using the web pages, the web browser transmits information related to the measurement values set or input by the user to the information processing device 10. The transmitted information includes, for example, information such as designated plants, evaluation target materials, and the measurement values of each factor item.

Furthermore, the control unit 212 may enable the execution of the functions provided by the evaluation indices calculation platform by executing an installed application related to the calculation of evaluation indices for a client. The control unit 212 has an acquisition unit 213, an analysis unit 214, and an output unit 215 in order to execute the processing related to the calculation of evaluation indices of the present disclosure on a client side.

The acquisition unit 213 acquires the data set or input by the user through the user interface 250, or sensing data of each factor item from sensors or the like.

The analysis unit 214 performs designated analysis on each measurement value. An example of the designated analysis is omics analysis. The analysis unit 214 may extract significant measurement values using a designated statistical method on the basis of the result of the analysis.

The output unit 215 outputs the measurement values of each factor item, which have been analyzed or extracted as significant data, to the information processing device 10 via the network communication interface 220. Note that the analysis unit 214 may be included in the information processing device 10, so that the calculation of evaluation indices of the present disclosure is performed by the information processing device 10.

<Data Example>

Figure 4 is a diagram showing an example of each factor item information (part 1) in the present disclosure. Each factor item information shown in Figure 4 includes each factor item that can be measured or analyzed in a laboratory. Furthermore, the factor items are classified into objectives (major categories), types (subcategory), and measurement/analysis (sub-subcategories), and they are associated with evaluation methods.

In the example shown in Figure 4, when the major category (objective) is "yield," the subcategory include "phenotype: high temperature" and "phenotype: normal temperature ." Furthermore, the subcategories "phenotype: high temperature" and "phenotype: normal temperature" include the sub-subcategories "aboveground weight," "aboveground length," "total biomass amount," "root weight ratio," and "root weight," and the analysis values or measurement values of each sub-subcategory are stored in the memory 130. The evaluation method for the factor items related to "yield" includes, for example, comparison values (ratios) in relation to the control. Furthermore, the "aboveground weight" and "root weight" can be measured through actual measurement using, for example, a microbalance, the "aboveground length" can be actually measured using, for example, a ruler, and the "total biomass amount" and "root weight ratio" can be obtained through calculation. For example, each measurement value is set or input by the user through the user interface 250.

Note that the phenotypes may be tested and evaluated for the sub-subcategories for each stress test. For example, each of the subcategories "phenotype: osmotic pressure," "phenotype: oxidation," "phenotype: drought," "phenotype: wounding," "phenotype: pests and diseases," and "phenotype: element" may be tested and evaluated for the sub-subcategories.

When the major category (objective) is "stress," the subcategory includes "expressed gene." The subcategory "expressed gene" includes "osmotic stress response," "pests and diseases stress response," "high-temperature stress response," "element stress response," "drought stress response," "wounding stress response" and "oxidative stress response." The analysis values of each sub-subcategory are obtained, for example, through omics analysis and stored in the memory 130. The evaluation method for the factor items related to "stress" includes, for example, the number of relevant items and a "weighted average based on P-Value." Furthermore, the sub-subcategories related to "expressed gene" are acquired, for example, through gene expression analysis, and each analysis value is set or input by the user, for example, through the user interface 250.

When the major category (objective) is "stimulus response," the subcategories include "plant hormone analysis: root" and "plant hormone analysis: leaf." The subcategories "plant hormone analysis: root" and "plant hormone analysis: leaf" include "salicylic acid," "auxin," "gibberellin (GA1)," "gibberellin (GA4)," "abscisic acid," "cytokinin (tZ)," "ethylene," "jasmonic acid," "strigolactone," "brassinosteroid," and "florigen." The analysis values or measurement values of each sub-subcategory are stored in the memory 130. The evaluation method for the factor items related to "stimulus response" includes, for example, measurement values obtained through analysis. The sub-subcategories related to "plant hormone analysis" are obtained, for example, through hormone analysis, and each analysis value is set or input by the user, for example, through the user interface 250.

When the major category "objective" is "nutrient absorption," the subcategory includes "nutrient element."

The subcategory "nutrient element" includes "N," "P," ... "Mn," etc., and the analysis values or measurement values of each sub-subcategory are stored in the memory 130. The evaluation method for the factor items related to "nutrient absorption" includes, for example, comparison values (ratios) in relation to the control. The sub-subcategories related to "nutrient element" are obtained through nutrient element analysis, and each analysis value is set or input by the user, for example, through the user interface 250.

Figure 5 is a diagram showing an example of each factor item information (part 2) in the present disclosure. Each factor item information shown in Figure 5 includes each factor item that can be measured or analyzed in an actual agricultural field test. In the example shown in Figure 5, when the major category (objective) is "soil improvement," the subcategories include "soil chemical analysis," "soil physical analysis," and "soil microbial community analysis."

The subcategory "soil chemical analysis" includes the sub-subcategories "N," "P," "K," and "other trace nutrient elements." The subcategory "soil physical analysis" includes the sub-subcategories "moisture content," "pH," "EC (electrical conductivity)," "permeability," and "hardness." The subcategory "soil microbial community analysis" includes the sub-subcategories "bacterial type (diversity)" and "bacterial containing ratio." The analysis values or measurement values of each sub-subcategory are stored in the memory 130. The evaluation method for the factor items related to "soil improvement" includes, for example, comparisons with the previous year or comparisons with target agricultural fields. Furthermore, the sub-subcategories related to "soil chemical analysis" are obtained through soil analysis based on sampling, the sub-subcategories related to "soil physical analysis" are obtained through soil analysis based on each sensor, and the sub-subcategories related to "soil microbial community analysis" are obtained through soil nutrient analysis based on sampling. Each analysis value is set or input by the user, for example, through the user interface 250.

When the major category "objective" is "stable production," the subcategories include "various stress tolerances" and "phenotype." The subcategory "various stress tolerances" includes the sub-subcategories "disease resistance," "high-temperature tolerance," "drought tolerance," and "salt tolerance." The subcategory "phenotype" includes the small item "yield." The analysis values or measurement values of each sub-subcategory are stored in the memory 130. The evaluation method for the factor items related to "stable production" includes, for example, comparisons with the previous year. Furthermore, the sub-subcategories related to "various stress tolerances" are obtained through observation or by using cultivation history data from production areas, and each measurement value is set or input by the user, for example, through the user interface 250. The sub-subcategory related to "phenotype" is obtained by using crop yields and cultivation history data from production areas, and each measurement value is set or input by the user, for example, through the user interface 250.

When the major category "objective" is "profitability," the subcategory includes "metabolite analysis." The subcategory "metabolite analysis" includes the sub-subcategories "sugar content," "functional components," "amino acids," "vitamins," "organic acids," and "bitterness." The analysis values or measurement values of each sub-subcategory are stored in the memory 130. The evaluation method for the factor items related to "profitability" includes, for example, comparison values (ratios) in relation to the control. Furthermore, the sub-subcategories related to "metabolite analysis" are obtained through metabolite analysis, and each analysis value is set or input by the user, for example, through the user interface 250.

<Weighting and Calculation Example of Evaluation Values>

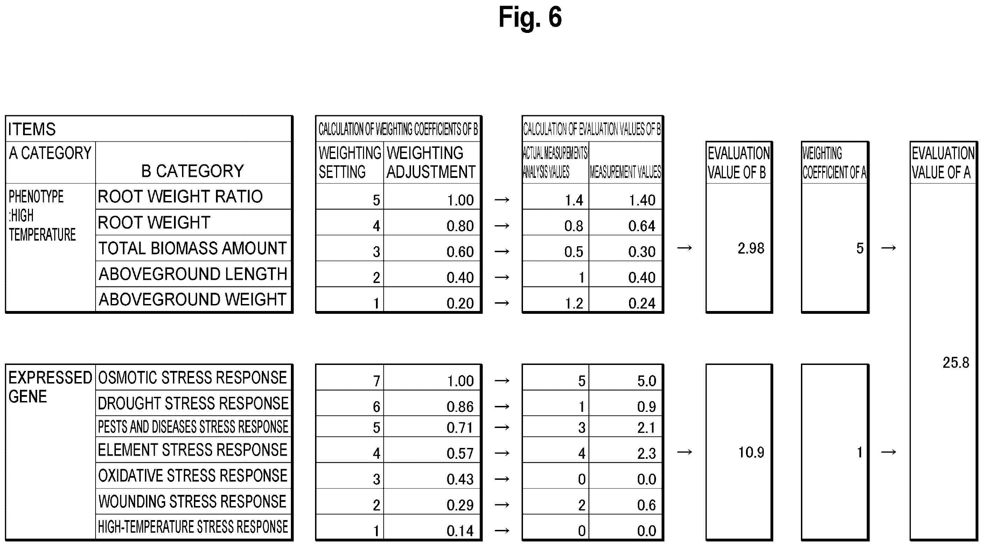

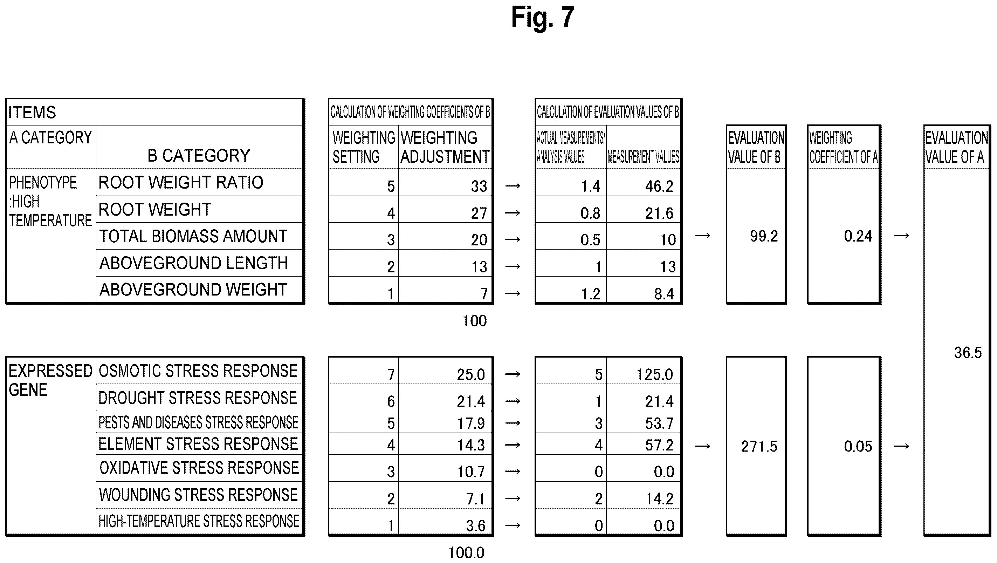

With reference to Figure 6 and Figure 7, the weighting of each factor item and the calculation of evaluation values in the present disclosure will be described using specific examples. First, regarding the weighting, an example of the weight-setting procedure performed by the setting unit 118 will be described.

1. The factor items of the major categories are prioritized on the basis of the degree of influence of materials on yield, the degree of influence on plant growth or stimuli, the sequence of physiological processes in plants, or the like.

2. The subcategories (category A) within the same major category are arranged in descending order of priority.

3. The sub-subcategories (category B) within the same subcategory are arranged in descending order of priority.

4. Weights are set for the subcategories.
Using the item with the highest priority among the subcategories as the reference point, the relative weights of the other subcategories are determined. The reference point is assumed to have the highest numerical value.

5. Weights are set for the sub-subcategories.

Using the item with the highest priority among the sub-subcategories as the reference point, the relative weights of the other sub-subcategories are determined. The reference point is assumed to have the highest numerical value.

The weight setting described above is provided as an example and is not limited to this example.

Figure 6 is a diagram showing an example of the weighting of each factor item and the calculation of evaluation values (case 1) in the present disclosure. In the example shown in Figure 6, for example, the sub-subcategories (category B) of the subcategory (category A) "phenotype: high temperature" are assigned weights of 5, 4, 3, 2, and 1 for "root weight ratio," "root weight," "total biomass amount," "aboveground length," and "aboveground weight," respectively. The priority described above is provided as an example. Each weight is adjusted to have a maximum value of 1.

The weighting unit 114 performs weighting on the measurement values (actual measurement values)/analysis values of each factor item of the sub-subcategories using weights. For example, the weighting unit 114 multiplies the actual measurement value 1.4 of the root weight ratio by a weight of 1 to calculate 1.4, and multiplies the actual measurement value 0.8 of the root weight by a weight of 0.8 to calculate 0.64. The weighting unit 114 performs the same processing on the other sub-subcategories in the same way.

Next, the weighting unit 114 adds all the values within the same subcategory together to create the evaluation value of the sub-subcategories (category B). For example, the weighting unit 114 creates an evaluation value of 2.98 for "phenotype: high temperature," and creates an evaluation value of 10.9 for "expressed gene."

Next, the weighting unit 114 weights the evaluation values of the subcategories using the weights of the subcategories. For example, when the weight of "phenotype: high temperature" is assumed to be 5 and the weight of "expressed gene" is assumed to be 1, the weighting evaluation value of "phenotype: high temperature" equals $2.98 \times 5$, and the weighting evaluation value of "expressed gene" equals $10.9 \times 1$.

The calculation unit 115 calculates the total sum of the weighted evaluation values to be used as the evaluation value of the material. For example, the calculation unit 115 calculates $2.98 \times 5 + 10.9 \times 1 = 25.8$ as the evaluation value of the material shown in Figure 6.

Figure 7 is a diagram showing an example of the weighting of each factor item and the calculation of evaluation values (case 2) in the present disclosure. In the example shown in Figure 7, the sub-subcategories (category B) of the subcategory (category A) "phenotype: high temperature" are adjusted so that the total of each weight equals 100.

The weighting unit 114 performs weighting on the measurement values (actual measurement values)/analysis values of each factor item of the sub-subcategories using weights. For example, the weighting unit 114 multiplies the actual measurement value 1.4 of the root weight ratio by a weight of 33 to calculate 46.2, and multiplies the actual measurement value 0.8 of the root weight by a weight of 27 to calculate 21.6. The weighting unit 114 performs the same processing on the other sub-subcategories in the same way.

Next, the weighting unit 114 adds all the values within the same subcategory together to create the evaluation value of the sub-subcategories (category B). For example, the weighting unit 114 creates an evaluation value of 99.2 for "phenotype: high temperature," and creates an evaluation value of 271.5 for "expressed gene."

Next, the weighting unit 114 weights the evaluation values of the subcategories using the weights of the subcategories. For example, when the weight of "phenotype: high temperature" is assumed to be 0.24 and the weight of "expressed gene" is assumed to be 0.05, the weighting evaluation value of "phenotype: high temperature" equals $99.2 \times 0.24$, and the weighting evaluation value of "expressed gene" equals $271.5 \times 0.05$.

The calculation unit 115 calculates the total sum of the weighted evaluation values to be used as the evaluation value of the material. For example, the calculation unit 115 calculates $99.2 \times 0.24 + 271.5 \times 0.05 = 36.5$ as the evaluation value of the material shown in Figure 6.

<Grading Example>

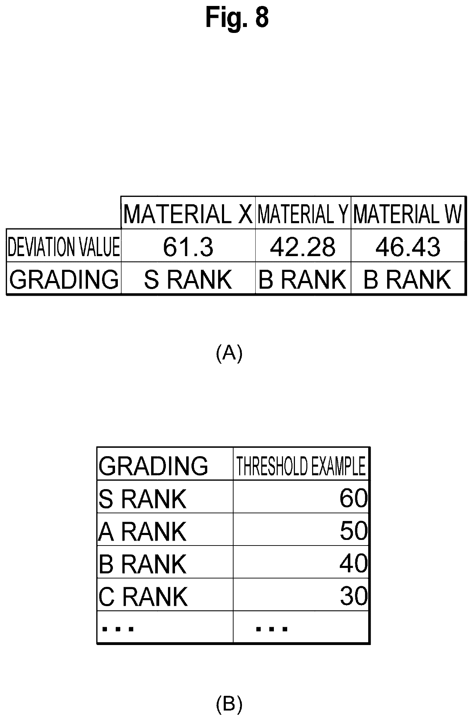

Figure 8 is a diagram showing an example of grading in the present disclosure. In the example shown in Figure 8, the calculation unit 115 calculates a standard deviation as the evaluation value of each material. Figure 8(A) shows the grading of each material, and Figure 8(B) shows an example of each threshold used for the grading.

In the example shown in Figure 8, the standard deviation of material X is assumed to be 61.3, the standard deviation of material Y is assumed to be 42.28, and the standard deviation of material W is assumed to be 45.43. Furthermore, when used for the grading, the thresholds are preset as 60 or higher for S rank, 50 or higher for A rank, and so on as conditions. In this case, the setting unit 118 assigns S rank because the standard deviation (61.3) of the material X is at least the threshold (60) for grading S, and assigns B rank because the standard deviation (42.28) of the material Y is at least the threshold (40) for grading B and below the threshold (50) for grading A.

<Processing Procedure>

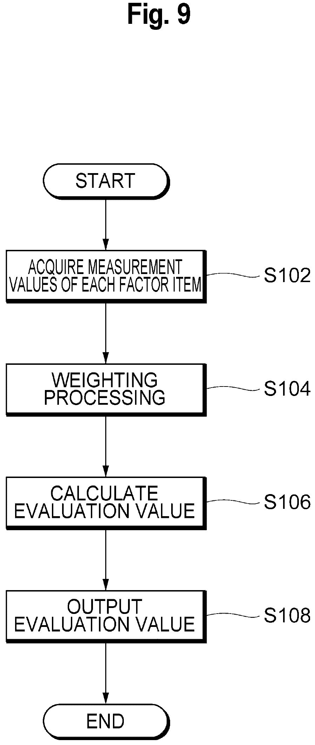

Next, each processing of the information processing system 1 will be described. Figure 9 is a sequence diagram showing an example of the evaluation processing performed by the information processing device 10 according to an embodiment of the present disclosure.

In step S102, the acquisition unit 113 of the information processing device 10 acquires the measurement values of one or a plurality of factor items related to biostimulant effect factors of a plant from the plant to which a material with the function of enhancing tolerance to abiotic stress has been applied.

In step S104, the weighting unit 114 of the information processing device 10 weights each measurement value acquired by the acquisition unit 113 using each weight.

In step S106, the calculation unit 115 of the information processing device 10 inputs each measurement value, which has been weighted by the weighting unit 114 using each weight, into a function related to the evaluation of the material to calculate the evaluation value of the material.

In step S108, the output unit 117 of the information processing device 10 outputs the evaluation value calculated by the calculation unit 115.

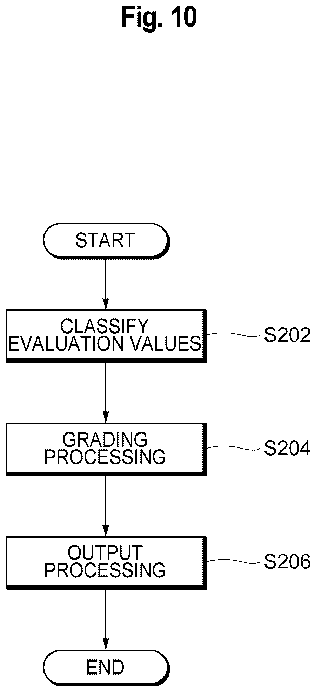

Figure 10 is a sequence diagram showing an example of the grading processing performed by the information processing device 10 according to an embodiment of the present disclosure. In the example shown in Figure 10, an example is described where grading is performed after classification processing. However, the classification processing is not necessarily involved in the grading processing, i.e., the grading processing may not follow the classification processing. Furthermore, the processing shown in Figure 10 may be executed when the evaluation value stored in the memory 130 is a prescribed value or more.

In step S202, the classification unit 116 of the information processing device 10 classifies each material using the evaluation values of each material. For example, the classification unit 116 clusters the evaluation values using a known clustering method and classifies the materials corresponding to the evaluation values. The classification unit 116 may classify the evaluation values for each raw material type of the main components of the materials and classify the materials corresponding to the evaluation values.

In step S204, the setting unit 118 of the information processing device 10 grades the materials within the clustered groups so that the groups with higher evaluation values are assigned higher ranks. Note that the setting unit 118 may perform the grading by comparing the evaluation values of the materials with the thresholds (see, for example, Figure 8).

In step S206, the output unit 117 of the information processing device 10 outputs the information related to the grading to an external device or display.

An embodiment of the present disclosure has been described in detail above, but the present disclosure is not limited to the above embodiment. Various modifications and alterations are possible within the scope of the claims. For example, regarding the processing executed by each of the information processing device 10 and 20, some of the processing may be handled by other information processing devices, or a plurality of information processing devices may be appropriately integrated so that all the processing is executed by a single device in the present disclosure. Furthermore, each of the information processing devices 10 and 20 may be managed by a single organization or by different organizations.

**Reference Signs List**

| | |
|---|---|
| 1 | Information processing system |
| 10, 20 | Information processing device |
| 110 | Processor |
| 112 | Control unit |
| 113 | Acquisition unit |
| 114 | Weighting unit |
| 115 | Calculation unit |
| 116 | Classification unit |
| 117 | Output unit |
| 118 | Setting unit |
| 130 | Memory |
| 210 | Processor |
| 230 | Memory |
| 212 | Control unit |
| 213 | Acquisition unit |
| 214 | Analysis unit |
| 215 | Output unit |

**Claims**

1. An information processing method, wherein one or a plurality of processors provided in an information processing device execute:

   acquiring measurement values of one or a plurality of factor items related to a biostimulant effect factor of a plant from the plant to which a material with a function of enhancing tolerance to abiotic stress has been applied;
   weighting each acquired measurement value of each factor item using each weight; and
   calculating an evaluation value of the material by inputting each measurement value weighted by each of the weights into a function related to evaluation of the material.

2. The information processing method according to claim 1, wherein each of the factor items includes at least one of a phenotype of the plant, an absorbed nutrient element, hormone analysis in response to a stimulus, and an expressed gene.

3. The information processing method according to claim 1, wherein each of the factor items includes at least one of soil chemical analysis, soil physical analysis, soil microbial community analysis, stress tolerance, or metabolite analysis.

4. The information processing method according to claim 1, wherein

each of the factor items is classified into a plurality of items through segmentation, and a weight is assigned to each item.

5. The information processing method according to claim 1, wherein,

when a plurality of different materials are applied to the plant,
the calculation includes calculating an evaluation value of each material for the plant, and
the one or the plurality of processors further execute classifying each of the materials using an evaluation value thereof.

6. The information processing method according to claim 5, wherein
the classification includes classifying, in response to a request for classification, materials that have evaluation values corresponding to a condition included in the request.

7. The information processing method according to claim 5, wherein
the classification includes classifying the materials using the evaluation values for each raw material type of the materials.

8. The information processing method according to claim 1, wherein,

when the evaluation value of the material satisfies a designated condition related to a recommendation,
the one or the plurality of processors further execute outputting information related to the material and recommendation information.

9. A non-transitory computer-readable recording medium having a program recorded thereon, wherein the program causes a computer to execute:

acquiring measurement values of one or a plurality of factor items related to a biostimulant effect factor of a plant from the plant to which a material with a function of enhancing tolerance to abiotic stress has been applied;
weighting each acquired measurement value of each factor item using each weight; and
calculating an evaluation value of the material by inputting each measurement value weighted by each of the weights into a function related to evaluation of the material.

10. An information processing device comprising:

an acquisition unit that acquires measurement values of one or a plurality of factor items related to a biostimulant effect factor of a plant from the plant to which a material with a function of enhancing tolerance to abiotic stress has been applied;
a weighting unit that weights each acquired measurement value of each factor item using each weight; and
a calculation unit that calculates an evaluation value of the material by inputting each measurement value weighted by each of the weights into a function related to evaluation of the material.

# Fig. 1

# Fig. 2

**Fig. 3**

20

230 MEMORY

270

250 USER INTERFACE

220 NETWORK COMMUNICATION INTERFACE

210 PROCESSOR

212 CONTROL UNIT

213 ACQUISITION UNIT

214 ANALYSIS UNIT

215 OUTPUT UNIT

# Fig. 4

| MAJOR CATEGORY | SUBCATEGORY | SUB-SUBCATEGORY | EVALUATION METHOD | |
|---|---|---|---|---|
| OBJECTIVE | CATEGORY A :TYPE | CATEGORY B :MEASUREMENT/ANALYSIS | 1 | 2 |
| YIELD | PHENOTYPE :HIGH TEMPERATURE | ABOVEGROUND WEIGHT | COMPARISON VALUES (RATIOS) IN RELATION TO CONTROL | |
| | | ABOVEGROUND LENGTH | | |
| | | TOTAL BIOMASS AMOUNT | | |
| | | ROOT WEIGHT RATIO | | |
| | | ROOT WEIGHT | | |
| | PHENOTYPE :NORMAL TEMPERATURE | ABOVEGROUND WEIGHT | COMPARISON VALUES (RATIOS) IN RELATION TO CONTROL | |
| | | . . . | | |
| STRESS | EXPRESSED GENE | OSMOTIC STRESS RESPONSE | THE NUMBER OF RELEVANT ITEMS | WEIGHTED AVERAGE BASED ON P-Value |
| | | HIGH-TEMPERATURE STRESS RESPONSE | | |
| | | PESTS AND DISEASES RESPONSE | | |
| | | ELEMENT STRESS RESPONSE | | |
| | | DROUGHT STRESS RESPONSE | | |
| | | WOUNDING STRESS RESPONSE | | |
| | | OXIDATIVE STRESS RESPONSE | | |
| STIMULUS RESPONSE | PLANT HORMONE ANALYSIS:ROOT | SALICYLIC ACID | MEASUREMENT VALUE | |
| | | AUXIN | | |
| | | GIBBERELLIN(GA1) | | |
| | | GIBBERELLIN(GA4) | | |
| | | ABSCISIC ACID | | |
| | | CYTOKININ(tZ) | | |
| | | ETHYLENE | | |
| | | JASMONIC ACID | | |
| | | STRIGOLACTONE | | |
| | | BRASSINOSTEROID | | |
| | | FLORIGEN | | |
| | PLANT HORMONE ANALYSIS:LEAF | SALICYLIC ACID | MEASUREMENT VALUE | |
| | | . . . | | |
| NUTRIENT ABSORPTION | NUTRIENT ELEMENT | N | COMPARISON VALUES (RATIOS) IN RELATION TO CONTROL | |
| | | P | | |
| | | . . . | | |
| | | Mn | | |

# Fig. 5

| MAJOR CATEGORY | SUBCATEGORY | SUB-SUBCATEGORY | EVALUATION METHOD |
|---|---|---|---|
| OBJECTIVE | CATEGORY A :TYPE | CATEGORY B :MEASUREMENT/ANALYSIS | |
| SOIL IMPROVEMENT | SOIL CHEMICAL ANALYSIS | N | COMPARISONS WITH PREVIOUS YEAR or COMPARISONS WITH TARGET AGRICULTURAL FIELDS |
| | | P | |
| | | K | |
| | | OTHER TRACE NUTRIENT ELEMENTS | |
| | SOIL PHYSICAL ANALYSIS | MOISTURE CONTENT | |
| | | pH | |
| | | EC(ELECTRICAL CONDUCTIVITY) | |
| | | PERMEABILITY | |
| | | HARDNESS | |
| | SOIL MICROBIAL COMMUNITY ANALYSIS | BACTERIAL TYPE (DIVERSITY) | |
| | | BACTERIAL CONTAINING RATIO | |
| STABLE PRODUCTION | VARIOUS STRESS TOLERANCES | DISEASE RESISTANCE | COMPARISONS WITH PREVIOUS YEAR (QUALITATIVE) |
| | | HIGH-TEMPERATURE TOLERANCE | |
| | | DROUGHT TOLERANCE | |
| | | SALT TOLERANCE | |
| | PHENOTYPE | YIELD | COMPARISONS WITH PREVIOUS YEAR |
| PROFITABILITY | SUGAR CONTENT | METABOLITE ANALYSIS | COMPARISON VALUES (RATIOS) IN RELATION TO CONTROL |
| | | FUNCTIONAL COMPONENTS | |
| | | AMINO ACIDS | |
| | | VITAMINS | |
| | | ORGANIC ACIDS | |
| | | BITTERNESS | |

# Fig. 6

**ITEMS**

| A CATEGORY | B CATEGORY | CALCULATION OF WEIGHTING COEFFICIENTS OF B | | | CALCULATION OF EVALUATION VALUES OF B | | | EVALUATION VALUE OF B | | WEIGHTING COEFFICIENT OF A | | EVALUATION VALUE OF A |
|---|---|---|---|---|---|---|---|---|---|---|---|---|
| | | WEIGHTING SETTING | WEIGHTING ADJUSTMENT | | ACTUAL MEASUREMENTS/ANALYSIS VALUES | MEASUREMENT VALUES | | | | | | |
| PHENOTYPE :HIGH TEMPERATURE | ROOT WEIGHT RATIO | 5 | 1.00 | → | 1.4 | 1.40 | | | | | | |
| | ROOT WEIGHT | 4 | 0.80 | → | 0.8 | 0.64 | | | | | | |
| | TOTAL BIOMASS AMOUNT | 3 | 0.60 | → | 0.5 | 0.30 | → | 2.98 | | 5 | → | |
| | ABOVEGROUND LENGTH | 2 | 0.40 | → | 1 | 0.40 | | | | | | 25.8 |
| | ABOVEGROUND WEIGHT | 1 | 0.20 | → | 1.2 | 0.24 | | | | | | |
| EXPRESSED GENE | OSMOTIC STRESS RESPONSE | 7 | 1.00 | → | 5 | 5.0 | | | | | | |
| | DROUGHT STRESS RESPONSE | 6 | 0.86 | → | 1 | 0.9 | | | | | | |
| | PESTS AND DISEASES STRESS RESPONSE | 5 | 0.71 | → | 3 | 2.1 | | | | | | |
| | ELEMENT STRESS RESPONSE | 4 | 0.57 | → | 4 | 2.3 | → | 10.9 | | 1 | → | |
| | OXIDATIVE STRESS RESPONSE | 3 | 0.43 | → | 0 | 0.0 | | | | | | |
| | WOUNDING STRESS RESPONSE | 2 | 0.29 | → | 2 | 0.6 | | | | | | |
| | HIGH-TEMPERATURE STRESS RESPONSE | 1 | 0.14 | → | 0 | 0.0 | | | | | | |

# Fig. 7

| ITEMS | | CALCULATION OF WEIGHTING COEFFICIENTS OF B | | CALCULATION OF EVALUATION VALUES OF B | | EVALUATION VALUE OF B | WEIGHTING COEFFICIENT OF A | EVALUATION VALUE OF A |
|---|---|---|---|---|---|---|---|---|
| A CATEGORY | B CATEGORY | WEIGHTING SETTING | WEIGHTING ADJUSTMENT | ACTUAL MEASUREMENTS/ ANALYSIS VALUES | MEASUREMENT VALUES | | | |
| PHENOTYPE :HIGH TEMPERATURE | ROOT WEIGHT RATIO | 5 | 33 → | 1.4 | 46.2 | | | |
| | ROOT WEIGHT | 4 | 27 → | 0.8 | 21.6 | | | |
| | TOTAL BIOMASS AMOUNT | 3 | 20 → | 0.5 | 10 | → 99.2 | 0.24 → | |
| | ABOVEGROUND LENGTH | 2 | 13 → | 1 | 13 | | | |
| | ABOVEGROUND WEIGHT | 1 | 7 → | 1.2 | 8.4 | | | |

100

| EXPRESSED GENE | OSMOTIC STRESS RESPONSE | 7 | 25.0 → | 5 | 125.0 | | | |
|---|---|---|---|---|---|---|---|---|
| | DROUGHT STRESS RESPONSE | 6 | 21.4 → | 1 | 21.4 | | | |
| | PESTS AND DISEASES STRESS RESPONSE | 5 | 17.9 → | 3 | 53.7 | | | |
| | ELEMENT STRESS RESPONSE | 4 | 14.3 → | 4 | 57.2 | → 271.5 | 0.05 → | |
| | OXIDATIVE STRESS RESPONSE | 3 | 10.7 → | 0 | 0.0 | | | |
| | WOUNDING STRESS RESPONSE | 2 | 7.1 → | 2 | 14.2 | | | |
| | HIGH-TEMPERATURE STRESS RESPONSE | 1 | 3.6 → | 0 | 0.0 | | | |

100.0

EVALUATION VALUE OF A: 36.5

# Fig. 8

|  | MATERIAL X | MATERIAL Y | MATERIAL W |
|---|---|---|---|
| DEVIATION VALUE | 61.3 | 42.28 | 46.43 |
| GRADING | S RANK | B RANK | B RANK |

(A)

| GRADING | THRESHOLD EXAMPLE |
|---|---|
| S RANK | 60 |
| A RANK | 50 |
| B RANK | 40 |
| C RANK | 30 |
| . . . | . . . |

(B)

# Fig. 9

```
         ┌─────────────┐
         │    START    │
         └──────┬──────┘
                │
                ▼
     ┌──────────────────────┐
     │  ACQUIRE MEASUREMENT  │────── S102
     │VALUES OF EACH FACTOR ITEM│
     └──────────┬───────────┘
                │
                ▼
     ┌──────────────────────┐
     │      WEIGHTING        │────── S104
     │      PROCESSING       │
     └──────────┬───────────┘
                │
                ▼
     ┌──────────────────────┐
     │      CALCULATE        │────── S106
     │   EVALUATION VALUE    │
     └──────────┬───────────┘
                │
                ▼
     ┌──────────────────────┐
     │        OUTPUT         │────── S108
     │   EVALUATION VALUE    │
     └──────────┬───────────┘
                │
                ▼
         ┌─────────────┐
         │     END     │
         └─────────────┘
```

# Fig. 10

```
        ( START )
            │
            ▼
┌───────────────────────┐
│       CLASSIFY        │ ── S202
│  EVALUATION VALUES    │
└───────────────────────┘
            │
            ▼
┌───────────────────────┐
│       GRADING         │ ── S204
│     PROCESSING        │
└───────────────────────┘
            │
            ▼
┌───────────────────────┐
│       OUTPUT          │ ── S206
│     PROCESSING        │
└───────────────────────┘
            │
            ▼
        (  END  )
```

# EP 4 553 498 A1

<table>
<tr><td colspan="2">INTERNATIONAL SEARCH REPORT</td><td colspan="2">International application No.<br><br>**PCT/JP2023/025087**</td></tr>
</table>

**A.    CLASSIFICATION OF SUBJECT MATTER**

*G01N 33/15*(2006.01)i; *G06Q 50/02*(2012.01)i
FI:    G01N33/15 C; G06Q50/02

According to International Patent Classification (IPC) or to both national classification and IPC

**B.    FIELDS SEARCHED**

Minimum documentation searched (classification system followed by classification symbols)

G01N33/15; G06Q50/02

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C.    DOCUMENTS CONSIDERED TO BE RELEVANT**

| Category* | Citation of document, with indication, where appropriate, of the relevant passages | Relevant to claim No. |
|---|---|---|
| A | WO 2018/147440 A1 (MENICON CO., LTD.) 16 August 2018 (2018-08-16)<br>entire text, all drawings | 1-10 |
| A | WO 2022/019114 A1 (SONY GROUP CORP.) 27 January 2022 (2022-01-27)<br>entire text, all drawings | 1-10 |
| A | JP 2009-131187 A (INST. OF PHYSICAL & CHEMICAL RESEARCH) 18 June 2009<br>(2009-06-18)<br>entire text, all drawings | 1-10 |
| A | WO 2013/151041 A1 (THE SHIZUOKA CHMBR. OF COMMERCE AND INDUSTRY) 10<br>October 2013 (2013-10-10)<br>entire text, all drawings | 1-10 |
| A | JP 2019-081746 A (KAO CORP.) 30 May 2019 (2019-05-30)<br>entire text, all drawings | 1-10 |

☐ Further documents are listed in the continuation of Box C.        ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br><br>**03 August 2023** | Date of mailing of the international search report<br><br>**05 September 2023** |
|---|---|
| Name and mailing address of the ISA/JP<br><br>**Japan Patent Office (ISA/JP)**<br>**3-4-3 Kasumigaseki, Chiyoda-ku, Tokyo 100-8915**<br>**Japan** | Authorized officer<br><br><br>Telephone No. |

Form PCT/ISA/210 (second sheet) (January 2015)

**INTERNATIONAL SEARCH REPORT**
Information on patent family members

International application No.

**PCT/JP2023/025087**

| Patent document cited in search report | | | Publication date (day/month/year) | Patent family member(s) | | | Publication date (day/month/year) |
|---|---|---|---|---|---|---|---|
| WO | 2018/147440 | A1 | 16 August 2018 | US | 2020/0060269 | A1 | |
| | | | | entire text, all drawings | | | |
| | | | | WO | 2018/147439 | A1 | |
| | | | | CN | 110392529 | A | |
| WO | 2022/019114 | A1 | 27 January 2022 | (Family: none) | | | |
| JP | 2009-131187 | A | 18 June 2009 | (Family: none) | | | |
| WO | 2013/151041 | A1 | 10 October 2013 | US | 2014/0364316 | A1 | |
| | | | | entire text, all drawings | | | |
| | | | | EP | 2759201 | A1 | |
| JP | 2019-081746 | A | 30 May 2019 | US | 2020/0045983 | A1 | |
| | | | | entire text, all drawings | | | |
| | | | | WO | 2018/159393 | A1 | |
| | | | | CN | 110381736 | A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2022066901 A